# EUROPEAN PATENT SPECIFICATION

(11) **EP 3 620 180 B1**
(45) Date of publication and mention of the grant of the patent: **07.08.2024**
(21) Application number: 18306181.1
(22) Date of filing: 07.09.2018
(51) Int. Cl.: A61B 90/70, A61L 2/18, A61B 1/12

(54) **DISINFECTING/STERILIZING MACHINE FOR MEDICALS APPARATUS SUCH AS ENDOSCOPES**
DESINFEKTIONS-/STERILISATIONSMASCHINE FÜR MEDIZINISCHE VORRICHTUNGEN WIE ETWA ENDOSKOPE
MACHINE DE DÉSINFECTION/STÉRILISATION POUR DES APPAREILS MÉDICAUX TELS QUE DES ENDOSCOPES

(43) Date of publication of application: 11.03.2020
(73) Proprietor: Ecolab USA, Inc., St. Paul, Minnesota 55102 (US)
(72) Inventor: AFFRE, Christian, 13190 ALLAUCH (FR); RUIZ, Sébastien, 13340 ROGNAC (FR); BERENGUER, Laurent, 13400 AUBAGNE (FR); PENDARIES, Eric, 13600 LA CIOTAT (FR)
(74) Representative: Plasseraud IP

(56) References cited:
- EP-A1- 2 925 373
- EP-A2- 1 787 731
- EP-B1- 2 925 373
- WO-A1-03/072146
- WO-A1-2018/104690
- WO-A2-2008/097430
- IT-A1- 201700 106 764
- JP-A- H05 317 234
- US-A1- 2005 012 281

## Description

The invention relates to a disinfecting/sterilizing machine for disinfecting/sterilizing medicals apparatus such as endoscopes, and also an installation comprising a wall separating two rooms such as a clean room and a dirty room, said installation comprising a machine for disinfecting/sterilizing medicals apparatus according to invention disposed through an opening of said wall.

### FIELD OF INVENTION

The field of the invention relates to the technical sector of machines relating to the disinfecting/sterilizing of rigid or flexible endoscopes. More particularly the invention relates to such machines that are configured to be disposed through an opening of a wall separating a clean room and a service room. The clean room is usually of clean controlled atmosphere, typically under slight over pressure,

A used endoscope can be inserted into the disinfecting/sterilizing chamber of the machine from the service room so that a cleaning cycle is implemented in said disinfecting/sterilizing chamber. Once the cleaning cycle is completed, the cleaned endoscope can be extracted from the other side of the wall, that is to say from the clean room.

### DESCRIPTION OF PRIOR ART

A first type of pass through disinfecting/sterilizing machine is known for disinfecting endoscopes which comprises : a frame; a casing covering the frame so as to define a tunnel having a first opening and a second opening which is opposite from the first opening, an upper wall superiorly delimiting the tunnel and a bottom wall inferiorly delimitating the tunnel. A first door is hinged to the frame and configured to obstruct the first opening of said tunnel when closed, and a second door is hinged to the frame and configured to obstruct the second opening of the tunnel when closed. A basket is dimensioned so as to receive one or more endoscopes to be disinfected/sterilized, and which is dimensioned so as to transit along the tunnel.

The upper wall, the bottom wall, two side walls and first and second door, when closed, define a closed chamber for disinfecting/sterilizing the endoscope arranged in the basket.

In use, the casing of the machine is installed in an air tight manner in an opening of a wall separating a clean room from a service room. Clean room atmosphere is controlled, typically under slight over pressure. Endoscopes are typically arranged in the basket from the service room (dirty room), the basket carrying the endoscope being inserted into the tunnel through the first opening. The first door is then closed to implement a cleaning cycle in the disinfecting chamber. During this cycle, cleaning liquids are drawn from canisters by pumps, said pumped liquids being conveyed into the disinfecting chamber to clean the outer surfaces and the inner surfaces of endoscope. Once the cycle is completed, the second door is opened so that the basket carrying the endoscope is extracted through the second opening: the cleaned endoscope is extracted from the clean room.

As explained in document WO 2014/083524 A1, a drawback of such disinfecting/sterilizing machine is the particular large size thereof, this size being mainly due to the dimensions of the chamber receiving the basket whose dimensions cannot be reduced under a certain limit as the basket and the endoscope in turn occupy a certain volume. WO2018/104690 or EP 1787 731 A2 are example of such first type of pass through disinfecting/sterilizing machine.

WO 2014/083524 A1 discloses a second type of pass through disinfecting/sterilizing machine that does not require a basket to carry the endoscope. The disinfecting/sterilizing machine disclosed in EP disinfecting/sterilising comprises a tunnel having a first opening and a second opening; an upper wall which delimits the tunnel ; a first lateral wall positionable at the first opening such as to obstruct the tunnel; a second lateral wall positionable at the second opening such as to obstruct the tunnel; a bath for receiving an endoscope to be disinfected/sterilised; a bath support; guide means for guiding the group comprising the bath support and the bath along the tunnel between; a first endrun in which the bath projects externally from the first opening of the tunnel such as to be accessible for inserting or extracting the endoscope; and a second endrun in which the bath projects externally from the second opening of the tunnel such as to be accessible such as to insert or extract endoscope.

The upper wall forms, with said bath, a closed chamber such as to disinfect/sterilize the at least an endoscope when the bath is internal of the tunnel. The first lateral wall , when at the first opening, is couplable alternatively and removably, with the frame, such as to be solidly constrained to the frame and obstruct the tunnel, or with the bath support such as to be solidly constrained to and be drawn by the group and enable the group to reach the first endrun. Similarly, the second lateral wall, when at the second opening, is alternatively and removably couplable; with the frame, such as to be solidly constrained to the frame and obstruct the tunnel, or with the bath support such as to be solidly constrained to and be drawn by the group and enable the group to reach the second endrun.

As shown on Fig 3A of WO 2014/083524 A1, when the bath is inside the tunnel, cleaning means are activated to wash and disinfect/sterilize the endoscope contained in the chamber. Cleaning means comprise hydraulic circuit which opens in the bath, and a final expulsion through as discharge of the bath. A cleaning cycle can be implemented causing cleaning liquids such as detergents to flow into the bath, with a final expulsion to the discharge. Even though not explicitly illustrated, nor described in document WO 2014/083524 A1, the person skilled in the art understands that flexible pipes are mandatory to convey cleaning solution such as detergent from the canister to said bath, because the canister are housed in the casing and of a fixed position.

Moreover, due to the fact that said bath assembly must slide from said first end-run in which the bath projects externally from the first opening, to said second-run in which the bath projects externally from the second opening, the travel between first end-run and second end run is necessarily large, corresponding at least as twice the length of said bath in the sliding direction. Therefore, the flexibles pipe joining the canister and the bath must be of an overabundant length so that said flexibles pipes do not interfere with such a large displacement. In practice, according to the finding of the inventor such overabundant length of said flexible pipe makes it very difficult to mechanically guide said flexible pipes when the bath support is displaced from first end-run to second end run (and vice versa). For instance, and according to inventors, the travel is too large to use guide-chain to provide a proper guiding of the flexible pipe(s) during this displacement of the bath group.

Failing to provide a proper guiding of the flexible pipes when the bath is displaced from first end-run to second end-run improves the risk of deterioration of the pipes (i.e by pinching) when the bath is displaced, and thus the risks of pipe's leaks. This risk is a first main drawback.

A second drawback inherent to the machined disclosed in WO 2014/083524 A1 is that the first lateral wall (resp. second lateral wall), when at the first opening (resp. second opening), is couplable alternatively and removably: with the frame, such as to be solidly constrained to the frame and obstruct the tunnel, or with the bath support such as to be solidly constrained to and be drawn by the group and enable the group to reach the first endrun (resp. second endrun).

Due to this design, and as described in WO 2014/083524 A1:
- a first complex mechanism comprising a first handle is mandatory to couple the first lateral wall with the frame, such as to be solidly constrained to the frame and obstruct the tunnel, and alternatively and removably with the bath support such as to be solidly constrained to and be drawn by the group and enable the group to reach the first endrun,
- a second complex mechanism comprising a second handle is mandatory to couple the second lateral wall with the frame, such as to be solidly constrained to the frame and obstruct the tunnel, and alternatively and removably with the bath support such as to be solidly constrained to and be drawn by the group and enable the group to reach the second endrun.

An operator who is in the service room needs to act on the first handle to removably couple the first lateral wall to the frame or to the bath support. Similarly an operator who is in the theater room needs to act on second handle so as to removably couple the second lateral wall to the frame or to the bath support.

Indeed, and according to WO 2014/083524 A1, in order to prevent air ingress between the two rooms (dirty room and clean room), and thus cross contamination, at least one of said first lateral wall (resp. said second lateral) must be coupled to the frame to obstruct in an air tight manner said first opening (reps. said second lateral) wall when said second wall (resp. first wall) is moved with said bath group.

According to the inventor's finding a second drawback of the disinfecting machine disclosed in WO 2014/083524 A1 is that first and second lateral wall needs a complex mechanism to couple them alternatively and removably with the frame or with the bath group. Such complex mechanisms are at the expense of the reliability: each time it is necessary to give access for inserting or extracting an endoscope from the clean room and the dirty room, the bath group must be slid up to first end-run, or upsecond end-run, (and vice versa), and thus, necessary to change the coupling of the first lateral wall (from bath group and frame, or vice versa), and/or the coupling of the second lateral wall (from bath group and frame, or vice versa)

The person skilled in the art knows a third type of pass through disinfecting machine from WASSENBURG^{®} which reference is WD440 PT. Such machine comprises a frame, a casing and a bath (or cleaning tank) which is of a fixed position relatively to the frame and casing. Access to the bath in order to insert or extract an endoscope from the service room ("*dirty room*") and access to the bath in in order to insert or extract an endoscope from the theater room ("*clean room*") is enabled thanks to a tilting lid that is configured to take three positions :
- an horizontal closed position wherein the lid obstructs tightly said upper opening of said bath so that a cleaning cycle is implemented in a closed position of the lid,
- a first tilted position wherein the lid opens the upper opening of the bath and is configured to give access to the bath in order to insert or extract an endoscope from the service room ( or "*dirty room*");
- a second tilted position wherein the lid opens bath's upper opening and is configured to give access to the bath in order to insert or extract an endoscope from the theater room (or "*clean room*").

A main drawback of this design is that the very same underneath surface of the lid passes from the dirty atmosphere of the service room when tilted in first tilted position (to give access to the bath in order to insert or extract an endoscope from the dirty room), to the clean atmosphere of the theater room when tilted in second tilted position (to give access to the bath in order to insert or extract an endoscope from the clean room). According to the finding of the inventor, the fact that this underneath surface of the lid passes from the atmosphere of the dirty room to the atmosphere of the clean room can lead to cross contamination between the two rooms. Indeed, if the central zone of the underneath surface of the lid forms an upper surface of the disinfecting chamber, and thus is disinfected by detergent during the implementation of a cleaning cycle, it remains a peripheral zone of said underneath surface of the lid which is not subjected to detergent during the cleaning cycle . This peripheral zone can be soiled during the insertion of a dirty endoscope, from the service room, and be tilted in a position wherein this soiled peripheral zone is in said clean room, without being disinfected by a cleaning cycle.

### SUMMARY OF THE INVENTION

The present invention aims to improve the situation described above.

One of the objects of the present invention is to propose a disinfecting/sterilizing machine for disinfecting/sterilizing medicals apparatus that limits the risk of deterioration of a flexible pipe conveying cleaning solution to the bath, compared to disinfecting machine of WO 2014/083524 A1.

Another aim of the invention is to propose a pass through disinfecting machine of a simpler design than the one of WO 2014/083524 A1 which guarantees long-term reliability.

Another aim of the invention is to propose a pass through disinfecting machine which will not cause problem of cross contamination between the dirty room and the clean room as described above.

To this end, the subject matter of the present invention relates to a disinfecting/sterilizing machine for disinfecting/sterilizing medicals apparatus such as rigid or flexible endoscopes, comprising:
- a frame and a casing being conformed to define a tunnel comprising at least an upper wall and at least one side wall, said tunnel having a first opening at a first end of the tunnel, and a second opening through the upper wall of said tunnel
- a tank assembly comprising a tank with a cavity configured to receive at least one medical apparatus, and a tank support, said cavity of said tank forming an upper tank opening,
- a guiding system comprising slides configured to guide the tank assembly between a first end position wherein the tank projects externally from the first opening such as to be accessible for inserting or extracting a medical apparatus, and a second end position wherein said tank is positioned inside said tunnel, said upper tank opening facing said second opening ,
- a movable lid comprising a cover body configured to obstruct the second opening in a first closed position of the lid, and to expose said second opening in a second opened position of the lid such that the tank positioned inside the tunnel at said second end position of said tank assembly is accessible for inserting or extracting a medical apparatus.
The travel of tank assembly to pass from first end position up to second end position is less than 200% of the length L of the tank in the direction of the slides.

Advantageously, the disinfecting/sterilizing machine is of pass-through type and can be positioned in air tight manner in an opening of a wall separating a service room ("*dirty room*") from a theater room ("*clean room*").

For example, the second opening of the tunnel closed by the lid is positioned in the service room while the first opening is oriented so that the tank assembly projects from said first opening in the clean room in said first position. An operator can insert an endoscope to be cleaned in said tank, through said second opening in the upper wall in said second opened position of the lid. After the lid is closed, a cleaning cycle can be implemented, for example by drawing cleaning liquids into the cavity of the tank. Once this cleaning cycle is completed, the tank assembly is slid from second position inside said tunnel up to first position wherein the tank projects from the first opening into the clean room, giving access so that the cleaned endoscope can be extracted from the tank.

The casing can comprise a fixed compartment receiving one or a plurality of canisters comprising disinfecting/sterilizing liquids, said machine comprising at least a cleaning circuit comprising a flexible pipe, and a pump, said circuit being configured to draw the cleaning liquid from the canister and to convey the liquid into the cavity of the tank in order to clean internal and/or external surfaces of the medical apparatus. One or a plurality of flexible pipe(s) joining the canister to the tank assembly are of an overabundant length so that the tank assembly is slideable from first end position to second end position, the flexible pipe not interfering with such a displacement.

The total travel of the tank assembly necessary to pass from said second position of the tank assembly, inside said tunnel, to said first position is of the order of the length of the tank assembly in the direction of the slides, that is to say around half of the travel necessary for the bath group to pass from first end run to said second end run in the machine according to document WO 2014/083524 A1. Such a reduced length travel makes it possible to guide properly the flexible pipe, by example using handling chain, and contrary to document WO 2014/083524 A1.

According to an embodiment, said movable lid comprises said cover body which is hinged to the casing so as to be displaced from said first closed position to said second open position. Said first closed position of the cover body may be locked, for instance using an electromagnetic lock. A programmable controller of the machine controls the electromagnetic lock so that said cover body stay locked when a cleaning cycle is implemented. A gas pressurized cylinder may be provided between the cover body and the frame/casing so that said pressurized cylinder automatically raises the cover body from said first closed position to second open position. Thus, once the electromagnetic lock is controlled to unlock the lid automatically opens second opening in the upper wall of said tunnel.

According to an embodiment, said tank assembly comprises a lateral wall fixedly securely to said tank assembly so as to be movable with the tank assembly and configured to obstruct said first opening wherein said tank assembly is inside the tunnel at second end position. A gasket (no shown) can be provide between said lateral wall and said first opening to makes sure said first opening is closed in an air tight manner when the tank assembly is in said first position. This provision prevents cross contamination, when the lid is opened, by forbidding air ingress through the casing from the dirty room up to the clean room, and vice versa.

According to these two last embodiments:
- said lateral wall configured to close or open first opening is secured and fixed to said tank assembly and
- the cover body of the lid configured to close or open said second opening is hinged to the frame/casing;
Therefore the machine according to invention does not need the complex mechanisms that are necessary in WO 2014/083524 A1 so that a wall is couplable alternatively and removably with the frame, such as to be solidly constrained to the frame and obstruct the tunnel, or with a moving support (said bath group) such as to be solidly constrained to and be drawn by the bath group. The reliability is improved.

According to an advantageous embodiment, said movable lid comprises - apart from said cover body - a seal device comprising a seal assembly with a seal plate and a gasket disposed along a periphery of the seal plate. The seal device is configured, in said first closed position of the lid, to seal in an fluid tight manner said upper tank opening, in at least one state of the seal device: therefore the internal surface of said cavity and the underneath surface of said seal plate defines a fluid tight sterilizing/disinfecting chamber.

Once closed in a fluid tight manner, the cleaning cycle may be implemented by the pump(s) so that cleaning liquid flowing from the canister is conveyed into the sterilizing/disinfecting chamber to clean the internal and/or the external surface of the medical apparatus. A programmable controller of the machine is programmed so that the seal device first seals in a fluid tight manner the disinfecting chamber, and before the cleaning liquid is brought into the disinfecting chamber by the pump(s).

According to an advantageous embodiment, said seal device comprises an actuator joining said seal plate and the cover body, configured, in said first closed position of said lid, to move the seal assembly between two positions, namely :
- a downward position, in particularly inside the cavity, so that the gasket seals directly the seal plate with the tank, in at said least one state of the gasket
- an upward position, above the cavity so that the tank assembly is free to slide from the second end position inside the tunnel to the first end position wherein the tank projects externally from the first opening. Such an advantageous embodiment makes sure that said seal assembly, in said upward position, does not interfere with the sliding of said tank assembly when the tank assembly is displaced from said second position inside said tunnel to said first position wherein said tank projects from first opening.

For instance, said actuator may be a pneumatic actuator, or an electric cylinder. Once the cleaning cycle is completed, said programmable controller controls said actuator so that said seal assembly is moved from downward position to said upward position, before allowing the sliding of said tank assembly from first second to first position, for example by controlling a second actuator configured to displace the tank assembly.

According to an advantageous embodiment, in said upward position of the seal assembly, said gasket is configured to seal in an air tight manner the upper wall with the seal plate in order to obstruct said second opening in an air tight manner, in at least one state of the gasket: such advantageous provision prevents air ingress between the two rooms through the casing (in particularly from the dirty room up to the clean room) when the tank assembly slides from said second position to said first position, and that said lateral wall does not obstruct anymore first opening.

The gasket may be provided on an edge of the seal plate, said gasket being an inflatable gasket, said seal device comprising a system to inflate (and deflate) said gasket to seal the gasket along:
a) the periphery of said cavity of the tank in the downward position of said seal assembly and/or,
b) the edge of second opening in the upper wall of said tunnel, in said upward position (Pb) of the seal assembly.

According to an embodiment, a programmable controller of the machine implements the chorological steps of a control sequence in order to pass said tank assembly from said second position up to said first position, comprising the steps of:
- deflating the inflated gasket of said seal assembly at said downward position,
- controlling the actuator so that said seal assembly passes from said downward position to said upward position,
- inflating said deflated gasket at said upward position, so that said seal assembly obstructs in an air tight manner the second opening in said upper wall of said tunnel, and afterward,
- controlling of a second actuator so that said tank assembly is displaced from second end position up to first end position.

According to an embodiment, the second end of the tunnel disposed opposed to said first opening is closed permanently, said casing comprising a user interface at said second end. For instance, user interface can comprise a screen such as a touch screen, and/or a controller keyboard.

According to an embodiment, the disinfecting/sterilizing machine may comprise a partition wall extending from said casing above the level of the second opening. Said partition wall is positioned between the first opening and the second opening, or positioned at said second opening. Said casing and said partition wall are intended to close a through opening in a wall separating from each other two rooms. Said partition wall may comprise a window allowing visual control.

Another object of said invention is to provide an installation comprising a wall separating two rooms such as a clean room and a dirty room, said wall comprising a through opening and a disinfecting/sterilizing apparatus according invention, said casing and said partition wall closing in an air tight manner the through opening in said wall, and wherein said tank assembly projects from a first side of the wall at said first end position so that a medical apparatus can be inserted or extracted from this first side, for example from said clean room, said tank assembly being positioned at said second end position on a second side of the wall so that a medical apparatus may be inserted or extracted through said second opening from said second side of the wall, for example from the dirty room.

According to an embodiment, the tank assembly projects at said first end position in a clean atmosphere, said machine comprising a device configured to draw air from said clean atmosphere and have the tunnel in over pressure with air from the clean atmosphere.

### BRIEF DESCRIPTION OF THE ACCOMPANYING FIGURES

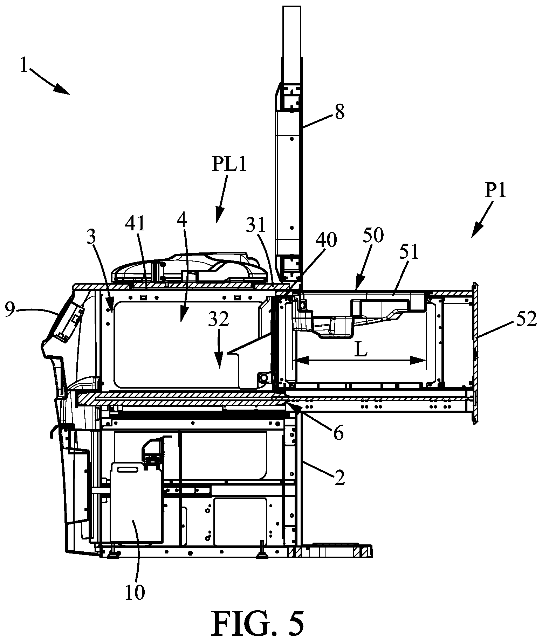

Other features and advantages of the present invention will emerge from the following description with reference to the accompanying FIGS. 1 to 7a, which illustrate an embodiment thereof with no limitative character and in which:
Figure 1 depicts a perspective view of an installation according to invention comprising a sterilizing/disinfecting machine disposed in an air tight manner in a through opening of a wall separating a service room and a theater room, the lid being in said first closed position, obstructing said second opening of said upper wall of said tunnel, said tank assembly being in said second position inside said tunnel, a lateral wall secured to said tank assembly obstructing the first opening of said tunnel.
Figure 2 illustrates the installation of figure 1, the cover body of said lid being disposed in said second position, giving access for inserting an endoscope in said tank through second opening, from said service room.
Figure 3 illustrates the installation of figure 3, the cover body of the lid being moved to said first closed position so that a cleaning cycle is implemented in the disinfecting/sterilizing chamber defined between the underneath surface of the lid and the cavity of said tank.
Figure 4 illustrates the installation of figure 3, after completion of a cleaning cycle, the tank assembly being moved from second position inside said tunnel up to first position wherein the tank assembly projects from first opening to give access for extracting said endoscope from the theater room.
Figure 5 is a cross-sectional view of figure 4.
Figure 6 is a detailed cross sectional view of figure 3 illustrating the disinfecting/sterilizing chamber, once closed to implement a cleaning cycle, the chamber being closed in a fluid tight manner by a seal device with a seal assembly comprising a seal plate and a gasket (not shown), said seal assembly being in a downward position inside said cavity of said tank so that the gasket, once inflated, seals directly the peripheral edge of the seal plate with the internal wall of the cavity of the tank.
Figure 6a is a detailed view of figure 6 illustrating said gasket that seals directly the seal plate with the lateral wall of the tank.
Figure 7 is a cross-sectional view of figure 4, said seal assembly being in an upward position so that tank assembly is free to slide and said gasket seals directly the peripheral edge of said seal plate with the edge of said second opening of said upper wall, preventing air leaks from service room to theater room through said tunnel.
Figure 7a is a detailed view of figure 7 illustrating in detail said gasket that seals directly the peripheral edge of said seal plate with the edge of said second opening of said upper wall.

### DETAILLED DESCRIPTION

The invention relates to a disinfecting/sterilizing machine 1 for disinfecting/sterilizing medicals apparatus, comprising:
- a frame 2 and a casing 3 being conformed to define a tunnel 4 comprising at least an upper wall 31 and at least one side wall (for instance two side walls 32, 33), said tunnel having a first opening 40 at a first end of the tunnel 4, and a second opening 41 through the upper wall 31 of said tunnel 4
- a tank assembly 5 comprising a tank 50 with a cavity configured to receive at least one medical apparatus, and a tank support, said cavity of said tank 50 forming an upper tank opening 51 (e.g., an opening defined and bounded in the X-Y plane that is accessible through movement in the Z-direction),
- a guiding system 6 comprising slides configured to guide the tank assembly 5 between a first end position P1 wherein the tank 50 projects externally from the first opening 40 such as to be accessible for inserting or extracting a medical apparatus, and a second end position P2 wherein said tank is positioned inside said tunnel 4, said upper tank opening 51 facing said second opening 41,
- a movable lid 7 comprising a cover body 70 configured to obstruct the second opening 41 in a first closed position PL1 of the lid, and to expose said second opening 41 in a second opened position PL2 of the lid such that the tank 50 positioned inside the tunnel 4 at said second end position P2 of said tank assembly 5 is accessible for inserting or extracting a medical apparatus.

Said movable lid 7 may comprise said cover body which is hinged to the casing so as to be displaced from said first closed position PL1 to said second open position PL2, and vice versa. Said machine may comprise a locking system, such as an electromagnetic lock, configured to lock said cover body in said first closed position PL1. The two parts of the electromagnetic lock are disposed respectively on the frame/casing and the cover body of the lid. A pressurized cylinder (not shown) may link said cover body 70 to the frame or casing, so that the restoring force of the pressurized cylinder, greater than the force of gravity on said lid, raises automatically said lid, from the first closed position PL1 to the second open position. Once the locking system unlocks said cover body 70, the lid 7 raises automatically.

Advantageously, said movable lid 7 can comprise, apart from said cover body 70, a seal device comprising a seal assembly with a seal plate 71 and a gasket 72 disposed along a periphery of the seal plate 71. Said seal device is configured, in said first closed position PL1 of the lid, to seal directly, in an fluid tight manner, said upper tank opening 51, in at least one state of the seal device : the internal surface of said cavity and the underneath surface of said seal plate 71 defines then the sterilizing disinfecting/sterilizing chamber.

A cleaning cycle is implemented in said disinfecting/sterilizing chamber, by conveying cleaning liquid, or water. The machine can comprise one or preferably a plurality of hydraulic circuit. For instance, the casing comprises a fixed compartment receiving one or a plurality of canisters 10 comprising disinfecting/sterilizing liquids, said machine comprising at least one cleaning circuit comprising a flexible pipe, and a pump, said cleaning circuit being configured to draw the disinfecting liquid from the canister and to convey the liquid into the cavity of the tank in order to clean internal and/or external surfaces of the medical apparatus. For instance a first cleaning circuit can provide cleaning liquid to clean the external surface of the medical apparatus. The medical apparatus can be immersed in an amount of cleaning liquid received in said tank. According to another embodiment, cleaning liquids can be sprayed onto said medical apparatus. A second cleaning circuit can provide liquid to clean the internal surface of the medical apparatus 1. The second circuit comprises a plug to connect the internal conduct with this second circuit.

The bottom of the tank may comprise a discharge coupled with a discharge circuit for emptying the liquids in said tank, such a discharge circuit comprising a flexible pipe.

Each of the flexible pipe belonging to the cleaning circuit and/or belonging to discharge circuit, can be of a overabundant length such that the tank assembly is slideable from first end position P1 to second end position P2, and vice versa, while canister are fixed into a compartment of said casing. A guiding system comprising a handling chain is advantageously configured to mechanically handle and guide the flexible pipe when said tank assembly 5 is displaced along the slides from first end position P1 to second end position P2, and vice versa. Flexible pipes are conveniently guided and maintained during this displacement, avoiding a risk of damage of said flexible pipes, for example by pinching.

Said tank assembly 5 can comprise a lateral wall 52 fixedly securely to said tank assembly so as to be movable with the tank assembly 5 and configured to obstruct said first opening 40 wherein said tank assembly 5 is inside the tunnel 4 at second end position P2. A gasket may be provided, on the periphery of said first opening, or secured to said wall so that the lateral wall 52 obstructs said first opening 40 in an air tight manner, when said tank assembly is at said second position P2. This provision makes sure no air ingress goes through said casing when the lid 7 is open, preventing cross contamination between the clean room and the dirty room.

The seal device may comprise an actuator 73 joining said seal plate 71 and the cover body 70, configured, in said first closed position PL1 of said lid, in said second position of the tank assembly 5, to move the seal assembly t between two positions:
- a downward position Pa so that the gasket seals the seal plate with the tank, in at said least one state of the gasket
- an upward position Pb, above the cavity so that the tank assembly is free to slide from the second end position P2 inside the tunnel to the first end position P1 wherein the tank assembly 5 projects externally from the first opening 40. Said actuator 73 may be a pneumatic actuator or an electric cylinder.

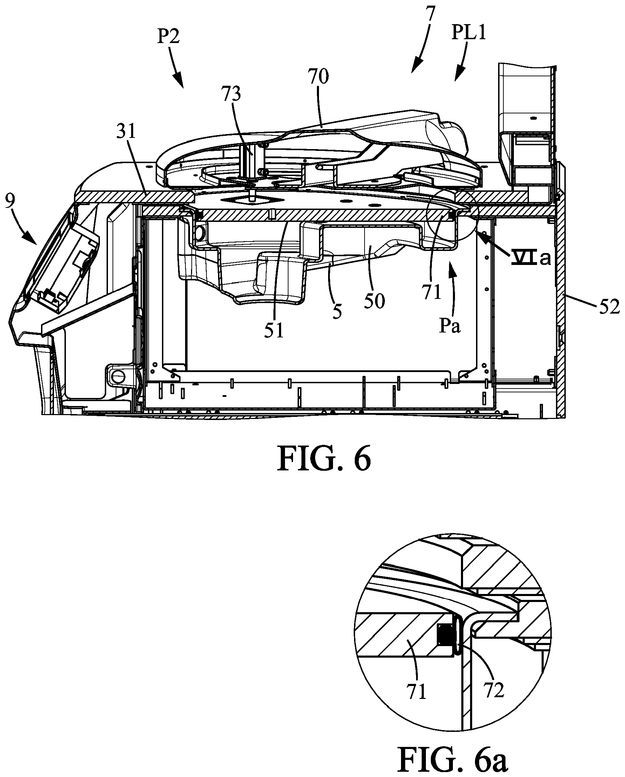

As illustrated on figure 6 in a non-limitative example, downward position Pa of said seal assembly makes sure said gasket may directly and properly seal in a fluid tight manner the upper tank opening. For instance, the gasket 72 is provided on an edge of the seal plate 71, said gasket being an inflatable gasket. Said seal device comprising a system to inflate said gasket to seal the gasket along the periphery of said cavity of the tank. As illustrated on figure 6, in this downward position Pa the seal assembly (including the seal plate 71 and gasket 72), is inside the cavity of said tank, thus preventing the sliding of said tank assembly.

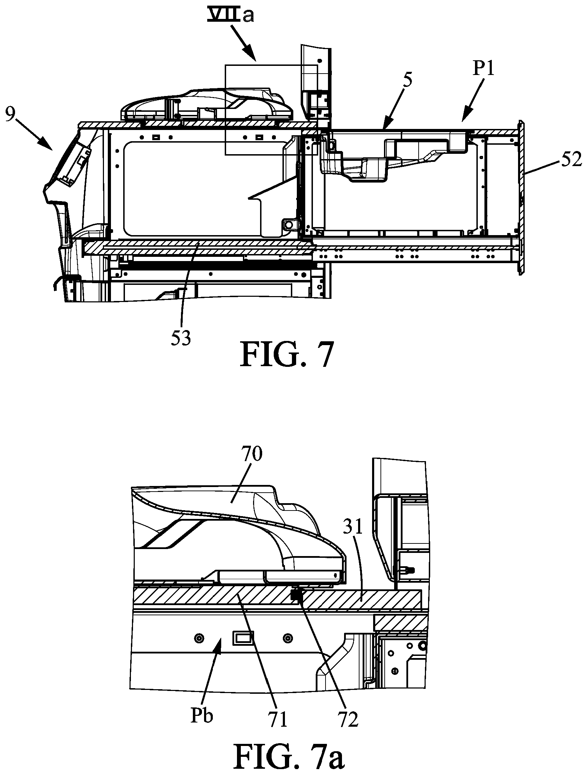

In said upward position Pb as illustrated on figure 7 and 7a, actuator 73 has moved said seal assembly above the cavity so that the tank assembly is free to slide from the second end position P2 inside the tunnel to the first end position P1 wherein the tank 50 projects externally from the first opening 40. Advantageously, as illustrated in figure 7a, in said upward position Pb of the seal assembly, said gasket 72 may be configured to seal in an air tight manner the upper wall 31 with the seal plate 71 in order to obstruct said second opening 41 in an air tight manner, in at least one state of the gasket. This provision makes sure that no air ingress goes through said second opening of said casing when tank assembly 5 is moved from second position P2 inside said tunnel up to first position P1, preventing cross contamination between the dirty room and the clean room.

The gasket 72 may be provided on an edge of the seal plate 71, comprising a peripheral groove. The peripheral groove receives a peripheral rib of said gasket 72. Said gasket may be an inflatable gasket, said seal device comprising a system to inflate said gasket to seal the gasket along:
a) the periphery of said cavity of the tank in the downward position Pa of said seal assembly and/or,
b) the edge of second opening in the upper wall 31 of said tunnel, in said upward position Pb of the seal assembly.

The inflatable gasket 72 may be inflated when the tank assembly 5 is in a static position, namely at said downward position Pa or at said upward position Pb. The inflatable gasket 72 is deflated before moving the seal assembly upward or downward.

Alternatively, according to an embodiment (not shown), in order to prevent air ingress when tank assembly 5 is moved up to first position P1, the lid can comprise a second seal device, with a (second) gasket configured to seal second opening, in said first closed position of the lid, and irrespective of the position of the seal plate of said (first) seal device.

The second end of the tunnel 4 disposed opposed to said first opening 40 is closed, said casing comprising a user interface 9 at said second end. This user interface may comprise a screen such as a touch screen and or a controller keyboard.

Said disinfecting/sterilizing machine can comprise a partition wall 8 extending from said casing above the level of the second opening 41, said partition wall being positioned between the first opening 40 and the second opening 41, or positioned at said second opening. Said casing 3 and said partition wall 8 are intended to close in an air tight manner a trough opening Op in a wall W separating from each other two separates rooms, for instance a service room ("*dirty room*") and a theater room ("*clean room*") typically of controlled atmosphere. Said partition wall 8 may comprise a window 80 giving visual access between the two room.

The invention also relates to an installation comprising a wall W separating two separate rooms for instance said service room and theater room, said wall W comprising a through opening Op and a disinfecting/sterilizing apparatus invention, said casing 3 and said partition wall closing in an air tight manner the through opening Op in said wall. Said tank assembly 5 projects from a first side of the wall W at said first end position P1 so that a medical apparatus can be inserted or extracted from this first side, for example from the clean room, said tank assembly being positioned at said second end position P2 on a second side of the wall W so that a medical apparatus may be inserted or extracted through said second opening 4 from said second side of the wall, for example from the service room.

The tank assembly can project at said first end position P1 in a clean atmosphere of the clean room. Advantageously, said machine can comprise a motorized fan device, configured to draw air from said clean atmosphere and have the tunnel 4 in overpressure with air from the clean atmosphere. Having the tunnel in a slight overpressure with the air from the clean room makes sure that no air ingress comes from the service room up to theater room when the tank assembly is slid from second end position up to first end position.

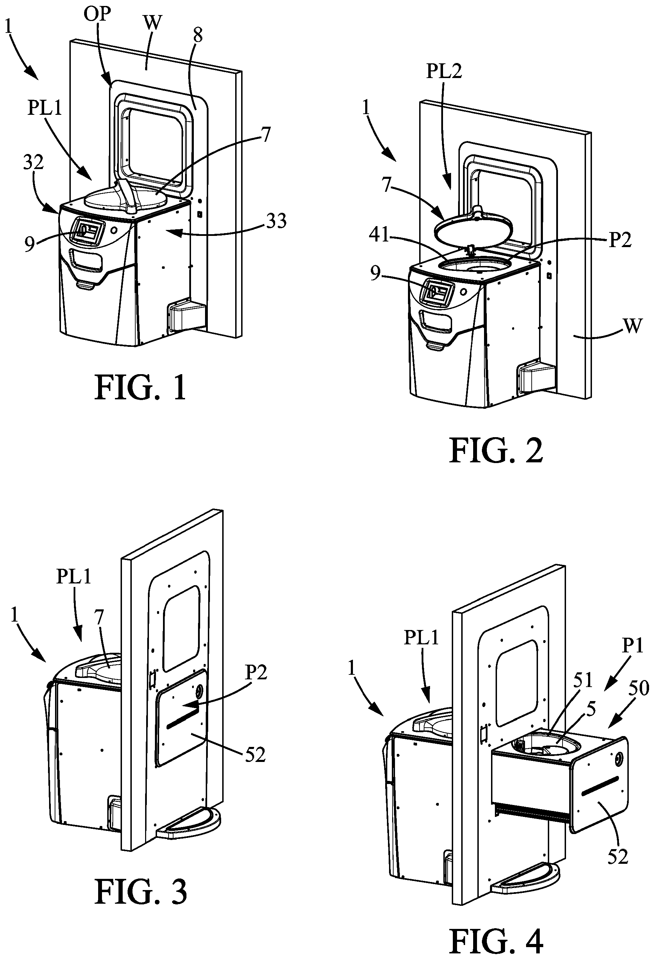

Such an installation as illustrated on the figures operates as follow : An operator from the service room acts on the user interface to unlock the lid which raises automatically upon action of the pressurized cylinder from first closed position PL1 (figure 1) up to second open position PL2 (figure 2). The operator then inserts a medical apparatus such as an endoscope into the tank, through the second opening 41 in the upper wall 31 of said tunnel 4. The operator connects the plug of said second cleaning circuit with internal conduit of said medical apparatus.

The operator pushes the lid downward against the restoring force of pressurized cylinder, so that the hinged cover body 70 closes again said second opening in the upper wall of the tunnel, then acts on user interface 9 to select a cleaning cycle. The cover body is then locked in said first closed position of the lid by the electromagnetic lock (figure 3). Then a programmable controller of said machine controls:
- said actuator 73 so that seal assembly (including seal plate 71) passes from upward position Pb to downward position Pa,
- the system to inflate said gasket 72 so that seal plate 71 and said internal wall of said cavity of the tank are sealed in a fluid tight manner
The programmable controller then controls the pumps (and typically vanes) of:
- the first cleaning circuit in order to fill the tank up to a determined level and thus immerse the medical apparatus with a cleaning liquid such as detergent, in order to clean the external surface of the medical apparatus,
- the second cleaning circuit so that cleaning liquid circulates through internal conduit of said medical apparatus.

The programmable controller may implement hereafter a step wherein a flushing liquid such as water is used to rinse the internal and external surfaces of the medical apparatus.

One the cleaning cycle is completed, and upon request from the service room (or the clean room), the tank may be displaced automatically from said second position P2, inside the tunnel up to first position P1 wherein the tank assembly 5 projects from first opening, so that the cleaned ondoscope may be extracted from said theater room. An actuator 53 (such as a pneumatic cylinder or an electric cylinder, or an electrical motor coupled with a driving belt or a gear rack) can drive the movement of the tank from first end position P1 to second end position P2, and vice versa.

For this purpose, the programmable controller of the machine implements the chorological steps of a control sequence comprising the steps of :
- deflating the inflated gasket 72 of said seal assembly at said downward position Pa,
- controlling the actuator 73 so that said seal assembly passes from downward position Pa to upward position Pb,
- inflating said deflated gasket 72 at said upward position Pb, so that said seal assembly obstructs in an air tight manner the second opening 41 in said upper wall 31 of said tunnel, and
- control of the (second) actuator 53 so that said tank assembly 5 is displaced from second end position P2 up to said first end position P1. In this last position, an operator from the theater room is able to extract the cleaned endoscope from the tank 50.

The total travel of the tank assembly necessary to pass from said second position of the tank assembly, inside said tunnel, to said first position can be of the order of the length of the tank assembly in the direction of the slides, that is to say around half of the travel necessary for the bath group to pass from first end run to said second end run in the machine according to document WO 2014/083524 A1. In WO2014/08524, this length is at least 200% of the length of the bath in the direction of the slides.

Such a reduced length travel according to invention makes it possible to guide properly the flexible pipe, by example using handling chain, and contrary to document WO 2014/083524 A1. The travel of tank assembly to pass from first end position up to second end position can be advantageously less than 200% of the length L of the tank in the direction of the slides, for example between 90% and 180%, in particularly between 90% and 150% of the length L of the tank in the direction of the slides. This travel can be between 300mm and 700mm, depending of the dimension of the tank.

### NOMENCLATURE

1. A disinfecting/sterilizing machine,
2. Frame,
3. Casing,
31. Upper wall,
32, 33. Side walls
4. Tunnel,
40. First opening (tunnel)
41. Second opening (tunnel),
5. Tank assembly,
50. Tank,
51. Upper tank opening,
52. Lateral wall,
53. Actuator,
6. Guiding system
7. (Movable) lid,
70. Cover body,
71. Seal plate (seal assembly),
72. Gasket (seal assembly),
73. Actuator,
8. Partition wall,
9. User Interface
W. Wall,
Op.Wall opening.
P1. First end position (tank assembly projects from first opening of the tunnel),
P2. Second end position (tank assembly inside the tunnel, the upper tank opening facing second opening of said upper wall),
PL1. First closed position (lid closing second opening)
PL2. Second open position (lid freeing second opening),
Pa. Downward position (seal assembly)
Pb. Upward position (seal assembly).
L. Length of the tank in the direction of the slides.

## Claims

1. A disinfecting/sterilizing machine (1) for disinfecting/sterilizing medicals apparatus, comprising:
- a frame (2) and a casing (3) being conformed to define a tunnel (4) comprising at least an upper wall (31) and at least one side walls (32, 33), said tunnel having a first opening (40) at a first end of the tunnel (4), and a second opening (41) through the upper wall (31) of said tunnel
- a tank assembly (5) comprising a tank (50) with a cavity configured to receive at least one medical apparatus, and a tank support, said cavity of said tank (50) having an upper tank opening (51),
- a guiding system (6) comprising slides configured to guide the tank assembly (5) between a first end position (P1) wherein the tank (50) projects externally from the first opening (40) such as to be accessible for inserting or extracting a medical apparatus, and a second end position (P2) wherein said tank is positioned inside said tunnel and said upper tank opening (51) faces said second opening (41),
- a movable lid (7) comprising a cover body (70) configured to obstruct the second opening (41) in a first closed position (PL1) of the lid, and to expose said second opening (41) in a second opened position (PL2) of the lid such that the tank (50) positioned inside the tunnel (4) at said second end position (P2) of said tank assembly (5) is accessible for inserting or extracting the at least one medical apparatus,
configured so that the travel of tank assembly to pass from first end position (P1) up to second end position (P2) is less than 200% of the length L of the tank in the direction of the slides.

2. A machine (1) according to claim 1 wherein said movable lid (7) comprises said cover body which is hinged to the casing so as to be displaced from said first closed position (PL1) to said second open position (PL2).

3. A machine (1) according to claim 1 or 2 wherein said movable lid (7) comprises, apart from said cover body (70), a seal device comprising a seal assembly with a seal plate (71) and a gasket (72) disposed along a periphery of the seal plate (71), said seal device being configured, in said first closed position (PL1) of the lid, to seal in an fluid tight manner said upper tank opening (51), in at least one state of the seal device, and wherein the internal surface of said cavity and the underneath surface of said seal plate (71) defines a sterilizing/disinfecting chamber.

4. A machine according to claim 3, wherein said seal device comprises an actuator (73) joining said seal plate (71) and the cover body (70), configured, in said first closed position (PL1) of said lid, to move the seal assembly comprising said seal plate and gasket between two positions :
- a downward position (Pa) so that the gasket seals the seal plate with the tank, in said at least one state of the gasket
- an upward position (Pb), above the cavity so that the tank assembly is free to slide from the second end position (P2) inside the tunnel to the first end position (P1) wherein the tank (50) projects externally from the first opening (40)

5. A machine according to claim 4 wherein said seal assembly is inside said cavity in said downward position (Pa) of said seal assembly.

6. A machine according to claim 4 or 5 wherein in said upward position of the seal assembly, said gasket is configured to seal in an air tight manner the upper wall (31) with the seal plate (71) in order to obstruct said second opening (41) in an air tight manner, in at least one state of the gasket.

7. A machine according to one of claim 4 to 6 wherein the gasket (72) is provided on an edge of the seal plate (71), said gasket being an inflatable gasket, said seal device comprising a system to inflate said gasket to seal the gasket along :
a) the periphery of said cavity of the tank in the downward position (Pa) of said seal assembly and/or,
b) the edge of second opening in the upper wall (31) of said tunnel, in said upward position (Pb) of the seal assembly.

8. A machine according to claims 6 and 7 wherein a programmable controller of the machine implements the chorological steps of a control sequence in order to pass said tank assembly (5) from said second position (P2) up to said first position (P1), comprising the steps of:
- deflating the inflated gasket (72) of said seal assembly at said downward position (Pa),
- controlling the actuator (73) so that said seal assembly passes from said downward position (Pa) to said upward position (Pb),
- inflating said deflated gasket (72) at said upward position (Pb), so that said seal assembly obstructs in an air tight manner the second opening (41) in said upper wall (31) of said tunnel, and afterward,
- controlling of a second actuator (53) so that said tank assembly (5) is displaced from said second end position (P2) up to first end position (P1).

9. A machine according to one of claims 1 to 8 wherein said tank assembly (5) comprises a lateral wall (52) fixedly securely to said tank assembly so as to be movable with the tank assembly (5) and configured to obstruct said first opening (40) wherein said tank assembly (5) is inside the tunnel (4) at second end position (P2).

10. A machine according to one of claims 1 to 9 wherein said casing comprises a fixed compartment receiving one or a plurality of canisters (10) comprising disinfecting/sterilizing liquids, said machine comprising at least cleaning circuit comprising a flexible pipe, and a pump, said circuit being configured to draw the disinfecting liquid from the canister and to convey the liquid into the cavity of the tank in order to clean internal and/or external surfaces of the medical apparatus, said flexible pipe joining the canister to the tank assembly, being of a overabundant length such that the tank assembly is slideable from first end position (P1) to second end position (P2) while the canister is fixed into said compartment, a guiding system comprising a handling chain being configured to mechanically handle and guide the flexible pipe when said tank assembly (5) is displaced along the slides from first end position (P1) to second end position (P2), and vice versa.

11. A machine according to one of claim 1 to 10 wherein a second end of the tunnel disposed opposed to said first opening (40) is closed, said casing comprising a user interface (9) at said second end.

12. A machine according to one of claims 1 to 11 comprising a partition wall (8) extending from said casing above the level of the second opening (41), said partition wall being positioned between the first opening (41) and the second opening (41), or positioned at said second opening, and wherein the said casing (3) and said partition wall (8) are intended to close a through opening (Op) in a wall (W) separating from each other two separates rooms.

13. A machine according to one of claims 1 to 12 wherein the travel of tank assembly to pass from first end position (P1) up to second end position (P2) is between 90% and 180% of the length L of the tank in the direction of the slides.

14. Installation comprising a wall (W) separating two separate rooms, said wall (W) comprising a through opening (Op) and a disinfecting/sterilizing machine according to claim 12, alone or in combination with claim 13, said casing (3) and said partition wall closing in an air tight manner the through opening (Op) in said wall, and wherein said tank assembly (5) projects from a first side of the wall (W) at said first end position (P1) so that a medical apparatus can be inserted or extracted from this first side, said tank assembly being positioned at said second end position (P2) on a second side of the wall (W) so that a medical apparatus may be inserted or extracted through said second opening (41) from said second side of the wall.

15. Use of the installation according to claim 14, wherein the tank assembly (5) projects at said first end position (P1) in a clean atmosphere, said machine comprising a device configured to draw air from said clean atmosphere and have the tunnel (4) in over pressure with air from the clean atmosphere.

## Patentansprüche

1. Desinfektions-/Sterilisationsmaschine (1) zum Desinfizieren/Sterilisieren von medizinischen Einrichtungen, umfassend:
- einen Rahmen (2) und ein Gehäuse (3), die angepasst sind, um einen Tunnel (4) zu definieren, umfassend mindestens eine obere Wand (31) und mindestens eine Seitenwand (32, 33), wobei der Tunnel eine erste Öffnung (40) an einem ersten Ende des Tunnels (4) und eine zweite Öffnung (41) durch die obere Wand (31) des Tunnels aufweist
- eine Tankanordnung (5), umfassend einen Tank (50) mit einem Hohlraum, der konfiguriert ist, um mindestens eine medizinische Einrichtung aufzunehmen, und einen Tankträger, wobei der Hohlraum des Tanks (50) eine obere Tanköffnung (51) aufweist,
- ein Führungssystem (6), umfassend Gleitschienen, die konfiguriert sind, um die Tankanordnung (5) zwischen einer ersten Endposition (P1), in der der Tank (50) nach außen aus der ersten Öffnung (40) derart hervorragt, dass er zum Einführen oder Herausnehmen einer medizinischen Einrichtung zugänglich ist, und einer zweiten Endposition (P2), in der der Tank innerhalb des Tunnels positioniert ist und die obere Tanköffnung (51) der zweiten Öffnung (41) zugewandt ist, zu führen,
- einen beweglichen Deckel (7), umfassend einen Abdeckkörper (70), der konfiguriert ist, um in einer ersten geschlossenen Position (PL1) des Deckels die zweite Öffnung (41) zu versperren, und in einer zweiten geöffneten Position (PL2) des Deckels die zweite Öffnung (41) derart freizulegen, dass der Tank (50), der innerhalb des Tunnels (4) an der zweiten Endposition (P2) der Tankanordnung (5) positioniert ist, zum Einführen oder Herausnehmen der mindestens einen medizinischen Einrichtung zugänglich ist,
so konfiguriert ist, dass der Weg der Tankanordnung, um von der ersten Endposition (P1) bis zu der zweiten Endposition (P2) befördert zu werden, weniger als 200 % der Länge L des Tanks in der Richtung der Gleitschienen beträgt.

2. Maschine (1) nach Anspruch 1, wobei der bewegliche Deckel (7) den Abdeckkörper umfasst, der an dem Gehäuse angelenkt ist, um aus der ersten geschlossenen Position (PL1) in die zweite offene Position (PL2) verlagert zu werden.

3. Maschine (1) nach Anspruch 1 oder 2, wobei der bewegliche Deckel (7) außer dem Abdeckkörper (70) eine Dichtungsvorrichtung umfasst, umfassend eine Dichtungsanordnung mit einer Dichtungsplatte (71) und einer Dichtung (72), die entlang eines Umfangs der Dichtungsplatte (71) eingerichtet ist, wobei die Dichtungsvorrichtung in der ersten geschlossenen Position (PL1) des Deckels konfiguriert ist, um in mindestens einem Zustand der Dichtungsvorrichtung die obere Tanköffnung (51) auf fluiddichte Weise abzudichten, und wobei die innere Oberfläche des Hohlraums und die Unteroberfläche der Dichtungsplatte (71) eine Sterilisations-/Desinfektionskammer definieren.

4. Maschine nach Anspruch 3, wobei die Dichtungsvorrichtung einen Aktuator (73) umfasst, der die Dichtungsplatte (71) und den Abdeckkörper (70) verbindet und der konfiguriert ist, um in der ersten geschlossenen Position (PL1) des Deckels die Dichtungsanordnung, umfassend die Dichtungsplatte und die Dichtung, zwischen zwei Positionen zu bewegen:
- eine nach unten gerichtete Position (Pa), so dass die Dichtung die Dichtungsplatte mit dem Tank abdichtet, in mindestens einem Zustand der Dichtung
- eine nach oben gerichtete Position (Pb) über dem Hohlraum, so dass die Tankanordnung ungehindert von der zweiten Endposition (P2) innerhalb des Tunnels zu der ersten Endposition (P1) gleiten kann, wobei der Tank (50) nach außen aus der ersten Öffnung (40) hervorragt.

5. Maschine nach Anspruch 4, wobei die Dichtungsanordnung innerhalb des Hohlraums in der nach unten gerichteten Position (Pa) der Dichtungsanordnung ist.

6. Maschine nach Anspruch 4 oder 5, wobei in der nach oben gerichteten Position der Dichtungsanordnung die Dichtung konfiguriert ist, um die obere Wand (31) mit der Dichtungsplatte (71) auf luftdichte Weise abzudichten, um die zweite Öffnung (41) in mindestens einem Zustand der Dichtung auf luftdichte Weise zu versperren.

7. Maschine nach einem der Ansprüche 4 bis 6, wobei die Dichtung (72) an einer Kante der Dichtungsplatte (71) bereitgestellt ist, die Dichtung eine aufblasbare Dichtung ist, die Dichtungsvorrichtung umfassend ein System, um die Dichtung aufzublasen, um die Dichtung abzudichten entlang:
a) dem Umfang des Hohlraums des Tanks in der nach unten gerichteten Position (Pa) der Dichtungsanordnung und/oder,
b) dem Rand der zweiten Öffnung in der oberen Wand (31) des Tunnels, in der nach oben gerichteten Position (Pb) der Dichtungsanordnung.

8. Maschine nach den Ansprüchen 6 und 7, wobei eine programmierbare Steuerung der Maschine die chronologischen Schritte einer Steuersequenz implementiert, um die Tankanordnung (5) von der zweiten Position (P2) bis zu der ersten Position (P1) zu befördern, umfassend die Schritte
- Entleeren der aufgeblasenen Dichtung (72) der Dichtungsanordnung an der nach unten gerichteten Position (Pa),
- Steuern des Aktuators (73), so dass die Dichtungsanordnung von der nach unten gerichteten Position (Pa) in die nach oben gerichtete Position (Pb) befördert wird,
- Aufblasen der entleerten Dichtung (72) an der nach oben gerichteten Position (Pb), so dass die Dichtungsanordnung die zweite Öffnung (41) in der oberen Wand (31) des Tunnels auf luftdichte Weise versperrt, und anschließend
- Steuern eines zweiten Aktuators (53), so dass die Tankanordnung (5) von der zweiten Endposition (P2) bis zu der ersten Endposition (P1) verlagert wird.

9. Maschine nach einem der Ansprüche 1 bis 8, wobei die Tankanordnung (5) eine laterale Wand (52) umfasst, die an der Tankanordnung fest gesichert ist, um mit der Tankanordnung (5) beweglich zu sein, und die konfiguriert ist, um die erste Öffnung (40) zu versperren, wobei die Tankanordnung (5) innerhalb des Tunnels (4) an der zweiten Endposition (P2) ist.

10. Maschine nach einem der Ansprüche 1 bis 9, wobei das Gehäuse ein befestigtes Fach umfasst, das einen oder eine Vielzahl von Kanistern (10), umfassend desinfizierende/sterilisierende Flüssigkeiten, aufnimmt, die Maschine umfassend mindestens einen Reinigungskreislauf, umfassend einen flexiblen Schlauch und eine Pumpe, wobei der Kreislauf konfiguriert ist, um die desinfizierende Flüssigkeit aus dem Kanister anzusaugen und die Flüssigkeit in den Hohlraum des Tanks zu liefern, um innere und/oder äußere Oberflächen der medizinischen Einrichtungen zu reinigen, wobei der flexible Schlauch, der den Kanister mit der Tankanordnung verbindet, eine übermäßige Länge hat, derart, dass die Tankanordnung von einer ersten Endposition (P1) zu einer zweiten Endposition (P2) gleitbar ist, während der Kanister in dem Fach befestigt ist, ein Führungssystem umfassend eine Handhabungskette, die konfiguriert ist, um den flexiblen Schlauch mechanisch zu handhaben und zu führen, wenn die Tankanordnung (5) entlang der Gleitschienen von der ersten Endposition (P1) zu der zweiten Endposition (P2) und umgekehrt verlagert wird.

11. Maschine nach einem der Ansprüche 1 bis 10, wobei ein zweites Ende des Tunnels, das der ersten Öffnung (40) gegenüberliegend eingerichtet ist, geschlossen ist, das Gehäuse umfassend eine Benutzerschnittstelle (9) an dem zweiten Ende.

12. Maschine nach einem der Ansprüche 1 bis 11, umfassend eine Trennwand (8), die sich von dem Gehäuse über die Ebene der zweiten Öffnung (41) hinaus erstreckt, wobei die Trennwand zwischen der ersten Öffnung (41) und der zweiten Öffnung (41) positioniert ist oder an der zweiten Öffnung positioniert ist, und wobei das Gehäuse (3) und die Trennwand (8) dazu bestimmt sind, eine Durchgangsöffnung (Op) in einer Wand (W) zu verschließen, die zwei getrennte Räume voneinander trennt.

13. Maschine nach einem der Ansprüche 1 bis 12, wobei der Weg der Tankanordnung von der ersten Endposition (P1) bis zu der zweiten Endposition (P2) zwischen 90 % und 180 % der Länge L des Tanks in der Richtung der Schlitten beträgt.

14. Anlage, umfassend eine Wand (W), die zwei getrennte Räume trennt, die Wand (W) umfassend eine Durchgangsöffnung (Op) und eine Desinfektions-/Sterilisationsmaschine nach Anspruch 12, allein oder in Kombination mit Anspruch 13, wobei das Gehäuse (3) und die Trennwand die Durchgangsöffnung (Op) in der Wand auf luftdichte Weise verschließen, und wobei die Tankanordnung (5) an der ersten Endposition (P1) von einer ersten Seite der Wand (W) so hervorragt, dass eine medizinische Einrichtung von dieser ersten Seite aus eingeführt oder herausgenommen werden kann, wobei die Tankanordnung an der zweiten Endposition (P2) auf einer zweiten Seite der Wand (W) so positioniert ist, dass eine medizinische Einrichtung durch die zweite Öffnung (41) von der zweiten Seite der Wand aus eingeführt oder herausgenommen werden kann.

15. Verwendung der Anlage nach Anspruch 14, wobei die Tankanordnung (5) an der ersten Endposition (P1) in eine saubere Atmosphäre hervorragt, die Maschine umfassend eine Vorrichtung, die konfiguriert ist, um Luft aus der sauberen Atmosphäre anzusaugen und den Tunnel (4) mit Luft aus der sauberen Atmosphäre unter Überdruck zu setzen.

## Revendications

1. Machine de désinfection/stérilisation (1) permettant la désinfection/stérilisation d'appareils médicaux, comprenant :
- un cadre (2) et une enveloppe (3) conformés pour définir un tunnel (4) comprenant au moins une paroi supérieure (31) et au moins une paroi latérale (32, 33), ledit tunnel ayant une première ouverture (40) au niveau d'une première extrémité du tunnel (4), et une seconde ouverture (41) à travers la paroi supérieure (31) dudit tunnel
- un ensemble réservoir (5) comprenant un réservoir (50) avec une cavité conçue pour recevoir au moins un appareil médical, et un support de réservoir, ladite cavité dudit réservoir (50) ayant une ouverture de réservoir supérieure (51),
- un système de guidage (6) comprenant des glissières conçues pour guider l'ensemble réservoir (5) entre une première position d'extrémité (P1) dans laquelle le réservoir (50) fait saillie vers l'extérieur de la première ouverture (40) de manière à être accessible pour l'insertion ou l'extraction d'un appareil médical, et une seconde position d'extrémité (P2) dans laquelle ledit réservoir est positionné à l'intérieur dudit tunnel et ladite ouverture supérieure de réservoir (51) fait face à ladite seconde ouverture (41),
- un couvercle mobile (7) comprenant un corps de couverture (70) conçu pour obstruer la seconde ouverture (41) dans une première position fermée (PL1) du couvercle, et pour exposer ladite seconde ouverture (41) dans une seconde position ouverte (PL2) du couvercle de telle sorte que le réservoir (50) positionné à l'intérieur du tunnel (4) au niveau de ladite seconde position d'extrémité (P2) dudit ensemble réservoir (5) est accessible pour l'insertion ou l'extraction de l'au moins un appareil médical,
conçu de sorte que la course de l'ensemble réservoir pour passer de la première position d'extrémité (P1) à la seconde position d'extrémité (P2) est inférieure à 200 % de la longueur L du réservoir dans la direction des glissières.

2. Machine (1) selon la revendication 1, dans laquelle ledit couvercle mobile (7) comprend ledit corps de couverture qui est articulé à l'enveloppe de manière à être déplacé de ladite première position fermée (PL1) à ladite seconde position ouverte (PL2).

3. Machine (1) selon la revendication 1 ou 2, dans laquelle ledit couvercle mobile (7) comprend, outre ledit corps de couverture (70), un dispositif d'étanchéité comprenant un ensemble d'étanchéité avec une plaque d'étanchéité (71) et un joint (72) disposé le long d'une périphérie de la plaque d'étanchéité (71), ledit dispositif d'étanchéité étant conçu , dans ladite première position fermée (PL1) du couvercle, pour sceller de manière étanche aux fluides ladite ouverture de réservoir supérieure (51), dans au moins un état du dispositif d'étanchéité, et dans laquelle la surface interne de ladite cavité et la surface inférieure de ladite plaque d'étanchéité (71) définissent une chambre de stérilisation/désinfection.

4. Machine selon la revendication 3, dans laquelle ledit dispositif d'étanchéité comprend un actionneur (73) reliant ladite plaque d'étanchéité (71) et le corps de couvercle (70), conçu, dans ladite première position fermée (PL1) dudit couvercle, pour déplacer l'ensemble d'étanchéité comprenant ladite plaque d'étanchéité et le joint entre deux positions :
- une position basse (Pa) de sorte que le joint assure l'étanchéité entre la plaque d'étanchéité et le réservoir, dans ledit au moins un état du joint
- une position haute (Pb), au-dessus de la cavité, de sorte que l'ensemble réservoir est libre de glisser de la seconde position d'extrémité (P2) à l'intérieur du tunnel vers la première position d'extrémité (P1), dans laquelle le réservoir (50) fait saillie vers l'extérieur de la première ouverture (40)

5. Machine selon la revendication 4, dans laquelle l'ensemble d'étanchéité se trouve à l'intérieur de ladite cavité dans la position basse (Pa) dudit ensemble d'étanchéité.

6. Machine selon la revendication 4 ou 5, dans laquelle, dans ladite position haute de l'ensemble d'étanchéité, ledit joint est conçu pour sceller de manière étanche à l'air la paroi supérieure (31) avec la plaque d'étanchéité (71) afin d'obstruer ladite seconde ouverture (41) de manière étanche à l'air, dans au moins un état du joint.

7. Machine selon l'une des revendications 4 à 6, dans laquelle le joint (72) est prévu sur un bord de la plaque d'étanchéité (71), ledit joint étant un joint gonflable, ledit dispositif d'étanchéité comprenant un système pour gonfler ledit joint afin de sceller le joint le long :
a) de la périphérie de ladite cavité du réservoir dans la position basse (Pa) dudit ensemble d'étanchéité et/ou,
b) du bord de la seconde ouverture dans la paroi supérieure (31) dudit tunnel, dans ladite position haute (Pb) de l'ensemble d'étanchéité.

8. Machine selon les revendications 6 et 7, dans laquelle un dispositif de commande programmable de la machine met en oeuvre les étapes chorologiques d'une séquence de commande afin de faire passer ledit ensemble réservoir (5) de ladite seconde position (P2) à ladite première position (P1), comprenant les étapes suivantes consistant à :
- dégonfler le joint gonflé (72) dudit ensemble d'étanchéité dans ladite position basse (Pa), - commander l'actionneur (73) de sorte que ledit ensemble d'étanchéité passe de ladite position basse (Pa) à ladite position haute (Pb),
- gonfler ledit joint dégonflé (72) au niveau de ladite position haute (Pb), de sorte que ledit ensemble d'étanchéité obstrue de manière étanche à l'air la seconde ouverture (41) dans ladite paroi supérieure (31) dudit tunnel, et par la suite,
- commander un second actionneur (53) de sorte que l'ensemble réservoir (5) est déplacé de ladite seconde position d'extrémité (P2) vers la première position d'extrémité (P1).

9. Machine selon l'une des revendications 1 à 8, dans laquelle ledit ensemble réservoir (5) comprend une paroi latérale (52) fixée de manière sûre à l'ensemble réservoir de manière à être mobile avec l'ensemble réservoir (5) et conçue pour obstruer ladite première ouverture (40), dans laquelle ledit ensemble réservoir (5) se trouve à l'intérieur du tunnel (4) dans la seconde position d'extrémité (P2).

10. Machine selon l'une des revendications 1 à 9, dans laquelle ladite enveloppe comprend un compartiment fixe recevant un ou plusieurs bidons (10) comprenant des liquides désinfectants/stérilisants, ladite machine comprenant au moins un circuit de nettoyage comprenant un tuyau flexible et une pompe, ledit circuit étant configuré pour aspirer le liquide désinfectant du bidon et pour acheminer le liquide dans la cavité du réservoir afin de nettoyer des surfaces internes et/ou externes de l'appareil médical, ledit tuyau flexible reliant le bidon à l'ensemble réservoir, étant d'une longueur surabondante de telle sorte que l'ensemble réservoir peut glisser de la première position d'extrémité (P1) à la seconde position d'extrémité (P2) tandis que le bidon est fixé dans ledit compartiment, un système de guidage comprenant une chaîne de manutention étant conçu pour manipuler et guider mécaniquement le tuyau flexible lorsque ledit ensemble réservoir (5) est déplacé le long des glissières de la première position d'extrémité (P1) à la seconde position d'extrémité (P2), et vice-versa.

11. Machine selon l'une des revendications 1 à 10, dans laquelle une seconde extrémité du tunnel opposée à ladite première ouverture (40) est fermée, ladite enveloppe comprenant une interface utilisateur (9) au niveau de ladite seconde extrémité.

12. Machine selon l'une des revendications 1 à 11 comprenant une paroi de séparation (8) s'étendant à partir de ladite enveloppe au-dessus du niveau de la seconde ouverture (41), ladite paroi de séparation étant positionnée entre la première ouverture (41) et la seconde ouverture (41), ou positionnée au niveau de ladite seconde ouverture, et dans laquelle ladite enveloppe (3) et ladite paroi de séparation (8) sont destinées à fermer une ouverture traversante (Op) dans une paroi (W) séparant l'un de l'autre deux emplacements distincts.

13. Machine selon l'une des revendications 1 à 12, dans laquelle la course de l'ensemble réservoir, pour passer de la première position d'extrémité (P1) à la seconde position d'extrémité (P2), est comprise entre 90 % et 180 % de la longueur L du réservoir dans la direction des glissières.

14. Installation comprenant une paroi (W) séparant deux emplacements distincts, ladite paroi (W) comprenant une ouverture traversante (Op) et une machine de désinfection/stérilisation selon la revendication 12, seule ou en combinaison avec la revendication 13, ladite enveloppe (3) et ladite paroi de séparation fermant de manière étanche à l'air l'ouverture traversante (Op) dans ladite paroi, et dans laquelle ledit ensemble réservoir (5) fait saillie d'un premier côté de la paroi (W) au niveau de ladite première position d'extrémité (P1) de sorte qu'un appareil médical peut être inséré ou extrait de ce premier côté, ledit ensemble réservoir étant positionné au niveau de ladite seconde position d'extrémité (P2) sur un second côté de la paroi (W) de sorte qu'un appareil médical peut être inséré ou extrait par le biais de ladite seconde ouverture (41) dudit second côté de la paroi.

15. Utilisation de l'installation selon la revendication 14, dans laquelle l'ensemble réservoir (5) fait saillie au niveau de ladite première position d'extrémité (P1) dans une atmosphère propre, ladite machine comprenant un dispositif conçu pour aspirer de l'air de ladite atmosphère propre et mettre le tunnel (4) en surpression avec de l'air de l'atmosphère propre.
